# EUROPEAN PATENT APPLICATION

(11) **EP 3 604 283 A1**
(43) Date of publication of application: **05.02.2020**
(21) Application number: 18777963.2
(22) Date of filing: 23.03.2018
(51) Int. Cl.: C07D 231/14

(54) **PRODUCTION METHOD FOR HALOGENATED PYRAZOLECARBOXYLIC ACID**

(30) Priority: 27.03.2017 JP 2017061336
(71) Applicant: AGC Inc., Chiyoda-ku Tokyo 100-8405 (JP)
(72) Inventor: SHIMIZU, Shota, Tokyo 100-8405 (JP); SAWAGUCHI, Masanori, Tokyo 100-8405 (JP)
(74) Representative: dompatent von Kreisler Selting Werner - Partnerschaft von Patent- und Rechtsanwälten mbB
(86) International application number: PCT/JP2018/011583
(87) International publication number: WO 2018/180944

(57) **Abstract**

The present invention provides a method capable of more simply and efficiently producing halogen-containing pyrazolecarboxylic acids useful as pharmaceutical or agrochemical intermediates, in a manner suitable for industrial production.

The present invention relates to a method of producing a compound represented by the formula (b), which comprises reacting a compound represented by the formula (a) with oxygen in the presence of a compound containing a transition metal atom to obtain the compound represented by the formula (b): 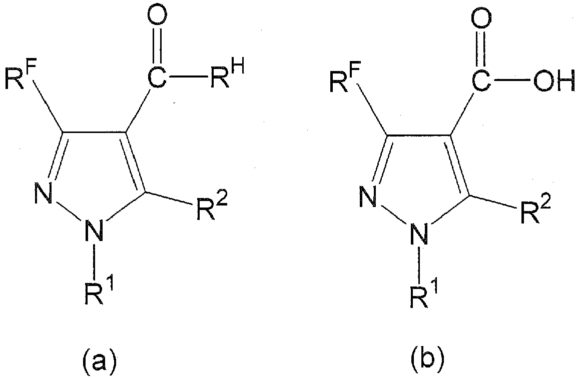 wherein each symbol is as described in the description.

## Description

### Technical Field

The present invention relates to a method of producing halogen-containing pyrazolecarboxylic acids (preferably fluorine-containing pyrazolecarboxylic acids) useful as pharmaceutical or agrochemical intermediates.

### Background Art

Halogen-containing pyrazolecarboxylic acids such as 3-difluoromethyl-1-methyl-1H-pyrazole-4-carboxylic acid and the like are intermediates useful for pyrazolylcarboxanilide fungicides.

Patent Document 1 discloses a method of reacting a compound represented by the following formula (a¹) with sodium hypochlorite in the presence of water to obtain a compound represented by the formula (b¹).

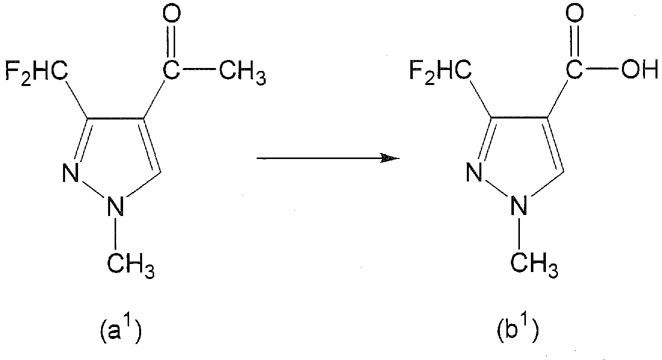

### Document List

### Patent Document

Patent Document 1: WO 2016/152886

### Summary of the Invention

### Problems to be Solved by the Invention

In the method using sodium hypochlorite, which is disclosed in Patent Document 1, an aqueous solution containing sodium hypochlorite is generally used.

In an aqueous solution mentioned above, the concentration of sodium hypochlorite cannot be increased, and therefore, a large amount of aqueous solution is required, which leads to additional work for wastewater treatment after reaction. Hence, use of an aqueous solution is not desirable from industrial aspect.

The aim of the present invention is to provide a method capable of more simply and efficiently producing halogen-containing pyrazolecarboxylic acids useful as pharmaceutical or agrochemical intermediates, in a manner suitable for industrial production.

### Means of Solving the Problems

The present inventors have conducted intensive studies, and found that the above-mentioned problems can be solved by use of oxygen in the presence of a specific compound.

Accordingly, the present invention encompasses the following inventions.

(1) A method of producing a compound represented by the below-mentioned formula (b), which comprises reacting the below-mentioned compound represented by the formula (a) with oxygen in the presence of a compound containing a transition metal atom to obtain the compound represented by the formula (b).
(2) The method of (1), wherein the compound containing a transition metal atom is a compound containing Mn, Co, Fe, Cu or Ni.
(3) The method of (1) or (2), wherein the compound containing a transition metal atom is a transition metal salt of nitric acid, a transition metal salt of sulfuric acid, a transition metal salt of acetic acid, a transition metal salt of carbonic acid, or a transition metal salt of phosphoric acid.
(4) The method of any one of (1) to (3), wherein the reaction of the compound represented by the formula (a) with oxygen is carried out in the presence of a carboxylic acid.
(5) The method of any one of (1) to (4), wherein the reaction of the compound represented by the formula (a) with oxygen is in the presence of nitric acid, an alkali metal salt of nitric acid, or an alkaline-earth metal salt of nitric acid.
(6) A method of producing a compound represented by the formula (b), which comprises reacting a compound represented by the formula (a) with oxygen in the presence of an alkali to obtain a compound represented by the formula (b).
(7) The method of (6), which further comprises, after the reaction of the compound represented by the formula (a) with oxygen, reacting the resulting compound with an acid.
(8) The method of (6) or (7), wherein the reaction of the compound represented by the formula (a) with oxygen is carried out in the presence of an organic solvent.
(9) The method of any one of (6) to (8), wherein the reaction of the compound represented by the formula (a) with oxygen is carried out in the presence of water.
(10) The method of any one of (6) to (9), wherein the alkali is sodium hydroxide or potassium hydroxide.

### Effect of the Invention

The present invention can provide a method capable of more simply and efficiently producing halogen-containing pyrazolecarboxylic acids useful as pharmaceutical or agrochemical intermediates, in a manner suitable for industrial production.

### Description of Embodiments

The present invention is explained below in detail. As used herein, numerical range shown by using "to" means the range including the numerical values described before and after "to" as minimum and maximum values.

The first embodiment of the present invention provides a method of producing a compound represented by the formula (b) (hereinafter, referred to as "compound (b)"), which comprises reacting a compound represented by the formula (a) (hereinafter, referred to as "compound (a)") with oxygen in the presence of a compound containing a transition metal atom (hereinafter, referred to as "compound M") to obtain compound (b). 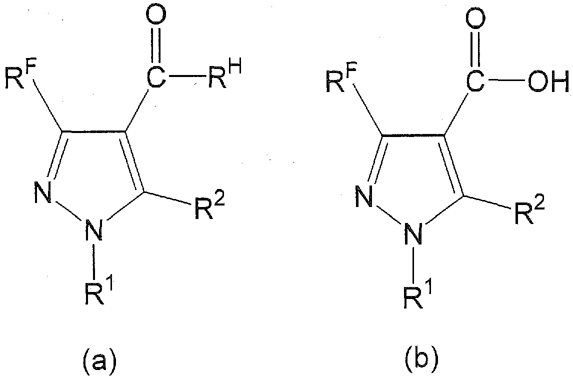 wherein R¹ is an alkyl group having 1 to 3 carbon atoms, preferably a methyl group.

R² is a hydrogen atom or a halogen atom. Specific examples of the halogen atom include a fluorine atom, a chlorine atom, a bromine atom and an iodine atom. R² is preferably a hydrogen atom.

R^{F} is a haloalkyl group having 1 to 3 carbon atoms. The haloalkyl group means a group wherein one or more hydrogen atoms of the alkyl group are replaced with halogen atoms. Specific examples of the halogen atom include a fluorine atom, a chlorine atom, a bromine atom and an iodine atom.

Specific examples of R^{F} include monohalomethyl groups (e.g., a fluoromethyl group, a chloromethyl group), dihalomethyl groups (e.g., a difluoromethyl group, a dichloromethyl group), trihalomethyl groups (e.g., a trifluoromethyl group, a dichlorofluoromethyl group, a chlorodifluoromethyl group), monohaloethyl groups (e.g., a 2-fluoroethyl group, a 2-chloroethyl group) and dihaloethyl groups (e.g., a 2,2-difluoroethyl group, a 2,2-dichloroethyl group).

R^{F} is preferably a monohalomethyl group, a dihalomethyl group or a trihalomethyl group, more preferably a dihalomethyl group or a trihalomethyl group, still more preferably a difluoromethyl group, a dichlorofluoromethyl group or a chlorodifluoromethyl group, particularly preferably a difluoromethyl group.

R^{H} is CₘH₂ₘ₊₁, and m is an integer of 1 to 3.

m is preferably 1 or 2, more preferably 1. That is, R^{H} is preferably CH₃ or C₂H₅, more preferably CH₃.

Compound M is preferably a compound containing Mn, Co, Fe, Cu or Ni, more preferably a compound containing Mn.

Examples of compound M include transition metal salts of nitric acid, transition metal salts of sulfuric acid, transition metal salts of acetic acid, transition metal salts of carbonic acid, transition metal salts of phosphoric acid, transition metals of hydroxide, transition metals of halide (chloride, bromide, fluoride, etc.), and transition metals of oxide. That is, compound M can also be referred to as an ionic compound containing a transition metal ion. Among them, preferred are transition metal salts of nitric acid, transition metal salts of sulfuric acid, transition metal salts of acetic acid, transition metal salts of carbonic acid, and transition metal salts of phosphoric acid, and more preferred are transition metal salts of nitric acid, and still more preferred are Mn(NO₃)₂, Co(NO₃)₂, Fe(NO₃)₃, Cu(NO₃)₂ and Ni(NO₃)₂, and particularly preferred is Mn(NO₃)₂, from the aspect of better conversion of the reaction. Compound M may be used in the form of a hydrate.

The amount of compound M to be used is preferably 0.001 mol equivalent or more relative to compound (a) from the aspect of conversion of the reaction, and preferably 2 mol equivalent or less, more preferably 0.001 to 2.0 mol equivalent, still more preferably 0.01 to 1.2 mol equivalent, relative to compound (a), from the aspect of inhibition of side reaction.

Compound M may be used alone or in combination of two or more kinds thereof.

The above-mentioned reaction is preferably carried out by introducing oxygen into a mixture of compound (a) and compound M.

In mixing compound (a) and compound M, both may be mixed in a batch, or one of compound (a) and compound M may be mixed to the other in divided portions.

In the first embodiment, the above-mentioned reaction is preferably carried out in the presence of a carboxylic acid. That is to say, the above-mentioned reaction is preferably carried out in the presence of compound M and a carboxylic acid. Use of a carboxylic acid further increases the conversion of the reaction.

The carboxylic acid is preferably an alkyl carboxylic acid having one or more a carboxy group. The alkyl carboxylic acid may be linear, branched or cyclic.

The alkyl carboxylic acid is preferably an alkyl carboxylic acid having 1 to 10 carbon atoms.

Specific examples of the alkyl carboxylic acid include acetic acid, propionic acid, butanoic acid, pentanoic acid, hexanoic acid, heptanoic acid, octanoic acid, pivalic acid, 3-methylbutanoic acid and cyclohexanecarboxylic acid. Among them, more preferred are acetic acid, pentanoic acid, hexanoic acid and octanoic acid, from the aspect of good yield of the reaction.

The combination of compound M and carboxylic acid is preferably a combination of one kind of compound M selected from the group consisting of Mn(NO₃)₂, Co(NO₃)₂, Fe(NO₃)₃, Cu(NO₃)₂ and Ni(NO₃)₂, and one kind of carboxylic acid selected from the group consisting of acetic acid, pentanoic acid, hexanoic acid and octanoic acid, more preferably a combination of Mn(NO₃)₂ and acetic acid, a combination of Mn(NO₃)₂ and pentanoic acid, a combination of Mn(NO₃)₂ and hexanoic acid, or a combination of Mn(NO₃)₂ and octanoic acid, from the aspect of yield.

The amount of the carboxylic acid to be used is preferably 1 to 1000-fold mass relative to compound (a) from the aspect of conversion of the reaction, more preferably 1.2 to 100-fold mass, still more preferably 2.0 to 30-fold mass, relative to compound (a), from the aspect of inhibition of side reaction.

The carboxylic acid may be used alone or in combination of two or more kinds thereof.

When the above-mentioned reaction is carried out in the presence of compound M and a carboxylic acid, the oxygen is preferably introduced into a mixture of compound (a), compound M and a carboxylic acid.

In the first embodiment, the above-mentioned reaction is preferably carried out in the presence of nitric acid, an alkali metal salt of nitric acid, or an alkaline-earth metal salt of nitric acid, from the aspect of easy acceleration of the reaction, and easy reaction progress in a short time. The reaction is more preferably carried out in the presence of compound M, a carboxylic acid and nitric acid, in the presence of compound M, a carboxylic acid and an alkali metal salt of nitric acid, or in the presence of compound M, a carboxylic acid and an alkaline-earth metal salt of nitric acid, still more preferably in the presence of compound M, a carboxylic acid and nitric acid from the aspect of easier acceleration of the reaction.

The alkali metal ion for the formation of an alkali metal salt of nitric acid is preferably Li⁺, Na⁺, K⁺, Rb⁺ or Cs⁺, more preferably Na⁺ or K⁺ from the aspect of good reactivity and easy availability.

Specific examples of the alkali metal salt of nitric acid include Li(NO₃), Na(NO₃), K(NO₃), Rb (NO₃) and Cs(NO₃).

The alkaline-earth metal ion for the formation of an alkaline-earth metal salt of nitric acid is preferably Mg²⁺, Ca²⁺ or Ba²⁺, more preferably Ca²⁺ from the aspect of good reactivity and easy availability.

Specific examples of the alkaline-earth metal salt of nitric acid include Mg(NO₃)₂, Ca(NO₃)₂ and Ba(NO₃)₂.

The amount of the nitric acid to be used is preferably 0.001 mol equivalent to 1.0 mol equivalent relative to compound (a) from the aspect of conversion of the reaction, more preferably 0.002 mol equivalent to 0.5 mol equivalent relative to compound (a) from the aspect of inhibition of side reaction.

The amount of the alkali metal salt of nitric acid or alkaline-earth metal salt of nitric acid to be used is preferably 0.001 mol equivalent to 1.0 mol equivalent relative to compound (a) from the aspect of conversion of the reaction, more preferably 0.002 mol equivalent to 0.5 mol equivalent relative to compound (a) from the aspect of inhibition of side reaction.

The combination of compound M, a carboxylic acid and nitric acid is preferably a combination of one kind of compound M selected from the group consisting of Mn(NO₃)₂, Co(NO₃)₂, Fe(NO₃)₃, Cu(NO₃)₂ and Ni(NO₃)₂, one kind of carboxylic acid selected from the group consisting of acetic acid, pentanoic acid, hexanoic acid and octanoic acid, and nitric acid, more preferably a combination of Mn(NO₃)₂, acetic acid and nitric acid, a combination of Mn(NO₃)₂, pentanoic acid and nitric acid, a combination of Mn(NO₃)₂, hexanoic acid and nitric acid, or a combination of Mn(NO₃)₂, octanoic acid and nitric acid, from the aspect of yield.

When the above-mentioned reaction is carried out in the presence of compound M, a carboxylic acid, and any of nitric acid, an alkali metal salt of nitric acid and an alkaline-earth metal salt of nitric acid, the oxygen is preferably introduced into a mixture of compound (a), compound M, a carboxylic acid, and any of nitric acid, an alkali metal salt of nitric acid and an alkaline-earth metal salt of nitric acid.

In the first embodiment, the oxygen is preferably used as mixed gas with the other gas.

The amount of the oxygen to be used is preferably 0.1 ml/min or more per 1 mol of compound (a) from the aspect of conversion of the reaction, and preferably 300 ml/min or less per 1 mol of compound (a) from the aspect of economic efficiency.

The reaction temperature in the first embodiment is preferably -20°C to +200°C, more preferably 0°C to 160°C, from the aspect of efficient reaction progress by inhibition of side reaction.

As a method of isolating compound (b) from the system after the completion of the reaction, for example, solvent extraction and crystallization can be employed.

The second embodiment of the present invention provides a method of producing compound (b), which comprises reacting compound (a) with oxygen in the presence of an alkali to obtain compound (b).

Specific examples of the alkali include lithium hydroxide, sodium hydroxide, potassium hydroxide, sodium methoxide, sodium ethoxide, sodium-tert-butoxide, potassium methoxide, potassium ethoxide and potassium-tert-butoxide. Among them, preferred are sodium hydroxide and potassium hydroxide, and particularly preferred is sodium hydroxide.

The above-mentioned reaction is preferably carried out by introducing oxygen into a mixture of compound (a) and an alkali.

In mixing compound (a) and an alkali, both may be mixed in a batch, or one of compound (a) and an alkali may be mixed to the other in divided portions.

The amount of the alkali to be used is preferably 0.8 mol equivalent or more relative to compound (a) from the aspect of conversion of the reaction, and preferably 10 mol equivalent or less, more preferably 0.8 to 10 mol equivalent, still more preferably 0.9 to 5.0 mol equivalent, relative to compound (a), from the aspect of inhibition of side reaction.

The alkali may be used alone or in combination of two or more kinds thereof.

In the second embodiment, the above-mentioned reaction is preferably carried out in the presence of water. That is to say, the above-mentioned reaction is preferably carried out in the presence of an alkali and water. Use of water further increases the conversion of the reaction.

The amount of the water to be used is preferably 0.01 mol equivalent or more relative to compound (a) from the aspect of conversion of the reaction, and preferably 500 mol equivalent or less, more preferably 0.01 to 500 mol equivalent, still more preferably 0.1 to 100 mol equivalent, relative to compound (a), from the aspect of inhibition of side reaction.

When the above-mentioned reaction is carried out in the presence of an alkali and water, the oxygen is preferably introduced into a mixture of compound (a), an alkali and water.

In the second embodiment, the above-mentioned reaction may be carried out in the presence of an organic solvent.

Examples of the organic solvent include protic organic solvents. Among them, preferred are tert-butanol, tert-amyl alcohol, acetonitrile, dimethylformamide, dimethylacetamide, dimethyl sulfoxide, N,N'-dimethylpropylene urea, sulfolane, 1,3-dimethyl-2-imidazolidinone and N-methyl-2-pyrrolidone, and more preferred are tert-butanol, tert-amyl alcohol, acetonitrile, dimethylformamide, dimethylacetamide, dimethyl sulfoxide and N-methyl-2-pyrrolidone, and particularly preferred are tert-butanol, tert-amyl alcohol, dimethylformamide, dimethyl sulfoxide and N-methyl-2-pyrrolidone. In this case, the solubility of compound (a) can be increased, which leads to efficient reaction progress.

When the above-mentioned reaction is carried out in the presence of an alkali and an organic solvent, the oxygen is preferably introduced into a mixture of compound (a), an alkali and an organic solvent.

In the second embodiment, the oxygen is preferably used as mixed gas with the other gas.

The amount of the oxygen to be used is preferably 0.1 ml/min or more per 1 mol of compound (a) from the aspect of conversion of the reaction, and preferably 300 ml/min or less per 1 mol of compound (a) from the aspect of economic efficiency.

The reaction temperature in the second embodiment is preferably -20°C to +200°C, more preferably 0°C to 160°C, from the aspect of efficient reaction progress by inhibition of side reaction.

In the second embodiment, after the completion of the reaction, the product obtained by the above-mentioned reaction is preferably reacted with an acid.

The product may be reacted with an acid after isolation. From the aspect of easy handling, the product is preferably reacted, directly as reaction mixture without isolation, with an acid.

Specific examples of the above-mentioned acid include sulfuric acid, hydrogen chloride, hydrochloric acid and nitric acid.

As a method of reacting the product with the above-mentioned acid, a method of mixing the product and the above-mentioned acid can be employed.

As a method of isolating compound (b) from the system after the completion of the reaction, for example, solvent extraction and crystallization can be employed.

Compound (b) is useful as intermediates for antimicrobials, fungicides, or bulk pharmaceuticals or agrochemicals, for example, useful as intermediates for the following antimicrobials, fungicides, or bulk pharmaceuticals or agrochemicals.

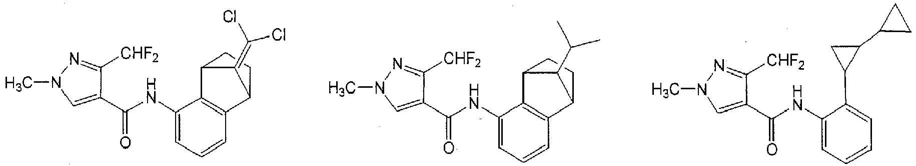

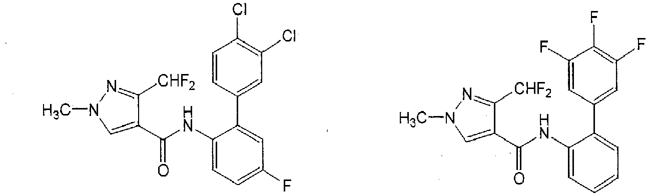

As a method of producing antimicrobials, fungicides, or bulk pharmaceuticals or agrochemicals using compound (b), the method described in Bioorganic & Medicinal Chemistry 24 (2016), p.317-341, or Chem. Rev. 114(2014), p.7079 can be employed. Specifically, for example, a method of reacting compound (b) with thionyl chloride to convert the carboxy group of compound (b) to the corresponding acid chloride group, and then reacting the resulting compound with the following amine compound can be employed.

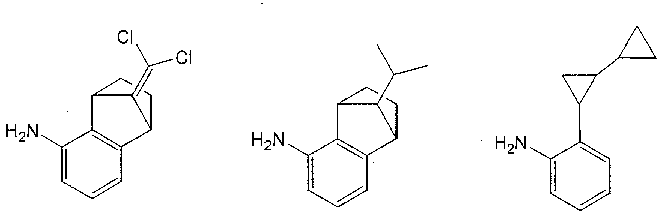

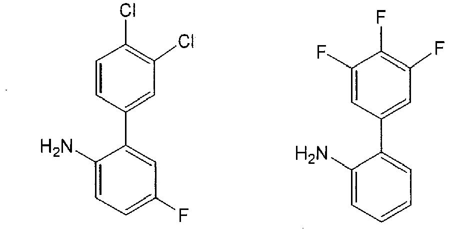

According to the production method of the present invention, the wastewater amount is reduced, and therefore, compound (b) can be more efficiently produced than in conventional art. That is to say, chloroalkanes (chloroform, etc.) generated as by-products in an oxidation reaction using sodium hypochlorite, as known in the conventional art, is not substantially generated in the production method of the present invention. Therefore, according to the production method of the present invention, antimicrobials, fungicides, or bulk pharmaceuticals or agrochemicals can be more efficiently produced than in conventional art.

### Examples

The present invention is explained in the following by referring to Examples, which are not to be construed as limitative.

Examples 1 to 13 are examples, and Example 14 is a comparative example. The results are shown in Table 1.

### [Example 1]

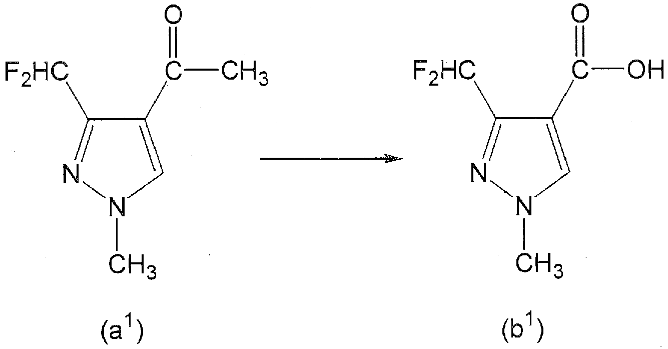

A mixture of compound (a¹) (1.0 g), manganese(II) nitrate hexahydrate (0.30 g, 20 mol% relative to compound (a¹)) and hexanoic acid (10 g) was heated to 100°C, and mixed gas (oxygen concentration: 21%) of oxygen and nitrogen was flowed into the mixture at 10 ml/min. The reaction progress was monitored by HPLC, and thereby, it was confirmed that the objective compound (b¹) was produced. The mixture was heated at 100°C for 8 hr, and subjected to quantitative analysis by HPLC. It was confirmed thereby that the yield of compound (b¹) was 95%. The reaction mixture was cooled to 25°C, and the precipitated solid was collected by filtration. The obtained solid was washed with chloroform and water to give compound (b¹) with purity 99% (HPLC analysis).

### [Example 2]

Compound (b¹) was obtained in the same manner as in Example 1, except that the manganese(II) nitrate hexahydrate was changed to iron(III) nitrate nonahydrate (0.44 g, 20 mol% relative to compound (a¹)).

### [Example 3]

Compound (b¹) was obtained in the same manner as in Example 1, except that the manganese(II) nitrate hexahydrate was changed to cobalt (II) nitrate hexahydrate (0.32 g, 20 mol% relative to compound (a¹)).

### [Example 4]

Compound (b¹) was obtained in the same manner as in Example 1, except that the manganese(II) nitrate hexahydrate was changed to nickel(II) nitrate hexahydrate (0.31 g, 20 mol% relative to compound (a¹)).

### [Example 5]

Compound (b¹) was obtained in the same manner as in Example 1, except that the manganese(II) nitrate hexahydrate was changed to copper(II) nitrate trihydrate (0.27 g, 20 mol% relative to compound (a¹)).

### [Example 6]

Compound (b¹) was obtained in the same manner as in Example 1, except that the hexanoic acid was changed to acetic acid. It was confirmed by quantitative analysis by HPLC that the yield of compound (b¹) was 97%.

### [Example 7]

Compound (b¹) was obtained in the same manner as in Example 1, except that the hexanoic acid was changed to pentanoic acid, and the heating time was changed from 8 hr to 6 hr. It was confirmed by quantitative analysis by HPLC that the yield of compound (b¹) was 97%.

### [Example 8]

Compound (b¹) was obtained in the same manner as in Example 1, except that the hexanoic acid was changed to octanoic acid, and the heating temperature of the mixture was changed from 100°C to 110°C, and the heating time was changed from 8 hr to 10 hr. It was confirmed by quantitative analysis by HPLC that the conversion of compound (a¹) was 52%, and the yield of compound (b¹) was 51%.

### [Example 9]

Compound (b¹) was obtained in the same manner as in Example 1, except that compound (a¹) (5.0 g), manganese(II) nitrate hexahydrate (1.98 g, 20 mol% relative to compound (a¹)) and octanoic acid (44 g) were used, and the heating time was changed from 8 hr to 27 hr. It was confirmed by quantitative analysis by HPLC that the conversion of compound (a¹) was 98%, and the yield of compound (b¹) was 90%.

### [Example 10]

A mixture of compound (a¹) (6.0 g), manganese(II) nitrate hexahydrate (1.65 g, 20 mol% relative to compound (a¹)), octanoic acid (53 g) and 70% nitric acid (0.24 g, 20 mol% relative to compound (a¹)) was heated to 100°C, and mixed gas (oxygen concentration: 21%) of oxygen and nitrogen was flowed into the mixture at 10 ml/min. The mixture was stirred at 100°C for 15 hr, and subjected to quantitative analysis by HPLC. It was confirmed thereby that the conversion of compound (a¹) was 97%, and the yield of compound (b¹) was 94%.

### [Example 11]

Compound (b¹) was obtained in the same manner as in Example 10, except that compound (a¹) (8.0 g), manganese(II) nitrate hexahydrate (0.52 g, 4 mol% relative to compound (a¹)), octanoic acid (70 g) and 70% nitric acid (0.63 g, 6 mol% relative to compound (a¹)) were used, and the heating time was changed from 15 hr to 17 hr. It was confirmed by quantitative analysis by HPLC that the conversion of compound (a¹) was 97%, and the yield of compound (b¹) was 97%.

### [Example 12]

A mixture of compound (a¹) (1.0 g), sodium hydroxide (1.1 g) and N-methyl-2-pyrrolidone (135 g) was heated to 40°C, and mixed gas (oxygen concentration: 21%) of oxygen and nitrogen was flowed into the mixture at 1500 ml/min. The reaction progress was monitored by HPLC, and thereby, it was confirmed that the objective compound (b¹) was produced. The mixture was heated at 40°C for 14 hr, and subjected to quantitative analysis by HPLC. It was confirmed thereby that the yield of compound (b¹) was 69%. The N-methyl-2-pyrrolidone was evaporated from the reaction solution under reduced pressure, and water was added thereto to dissolve the solid. Chloroform was added thereto, and the mixture was subjected to liquid separation. After liquid separation, sulfuric acid was added to the aqueous layer, and the precipitated solid was collected by filtration for recovery. The solid was washed with water, and subjected again to analysis. It was confirmed thereby that the HPLC purity of compound (b¹) was 99%.

### [Example 13]

A mixture of compound (a¹) (1.0 g), sodium hydroxide (0.92 g), N-methyl-2-pyrrolidone (135 g) and water (0.38 g, 4 mol equivalent relative to compound (a¹)) was heated to 40°C, and mixed gas (oxygen concentration: 21%) of oxygen and nitrogen was flowed into the mixture at 100 ml/min. The reaction progress was monitored by HPLC, and thereby, it was confirmed that the objective compound (b¹) was produced. The mixture was heated at 40°C for 23 hr, and subjected to quantitative analysis by HPLC. It was confirmed thereby that the yield of compound (b¹) was 80%. The mixture was heated at 70°C for 3 hr, and subjected again to quantitative analysis by HPLC. It was confirmed thereby that the yield of compound (b¹) was 83%.

### [Example 14]

A mixture of compound (a¹) and hexanoic acid was heated to 100°C, and mixed gas (oxygen concentration: 21%) of oxygen and nitrogen was flowed into the mixture at 10 mL/min. The reaction progress was monitored by HPLC, however, the production of the objective compound (b¹) was not observed exceeding detection limit.

**Table 1**

| | compound M | | acid or alkali | temperature | heating time | yield |
|---|---|---|---|---|---|---|
| | kind | [mol%] | kind | [°C] | [hr] | [%] |
| Ex.1 | Mn (NO₃)₂ | 20 | hexanoic acid | 100 | 8 | 95 |
| Ex.2 | Fe (NO₃)₃ | 20 | hexanoic acid | 100 | 8 | detected |
| Ex.3 | Co (NO₃) ₂ | 20 | hexanoic acid | 100 | 8 | |
| Ex.4 | Ni (NO₃)₂ | 20 | hexanoic acid | 100 | 8 | |
| Ex.5 | Cu (NO₃)₂ | 20 | hexanoic acid | 100 | 8 | |
| Ex.6 | Mn (NO₃)₂ | 20 | acetic acid | 100 | 8 | 97 |
| Ex.7 | Mn (NO₃)₂ | 20 | pentanoic acid | 100 | 6 | 97 |
| Ex.8 | Mn (NO₃) ₂ | 20 | octanoic acid | 110 | 10 | 51 |
| Ex.9 | Mn (NO₃)₂ | 20 | octanoic acid | 100 | 27 | 90 |
| Ex.10 | Mn (NO₃)₂ | 20 | octanoic acid nitric acid | 100 | 15 | 94 |
| Ex.11 | Mn(NO₃)₂ | 4 | octanoic acid nitric acid | 100 | 17 | 97 |
| Ex.12 | - | - | NaOH | 40 | 14 | 69 |
| Ex.13 | - | - | NaOH | 40 | 23 | 80 |
| Ex.14 | - | - | hexanoic acid | 100 | - | not detected |

### Industrial Applicability

As is clear from the above results, according to the production method of the present invention, halogen-containing pyrazolecarboxylic acids useful as pharmaceutical or agrochemical intermediates, can be produced more simply and efficiently.

This application is based on patent application No. 2017-061336 filed on March 27, 2017 in Japan, the contents of which are encompassed in full herein.

## Claims

1. A method of producing a compound represented by the formula (b), which comprises reacting a compound represented by the formula (a) with oxygen in the presence of a compound containing a transition metal atom to obtain a compound represented by the formula (b); 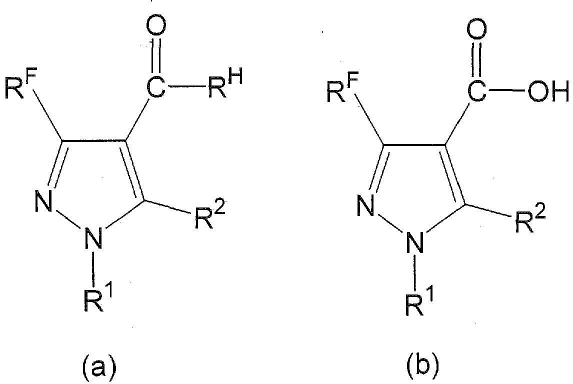 wherein
R¹ is an alkyl group having 1 to 3 carbon atoms,
R² is a hydrogen atom or a halogen atom,
R^{F} is a haloalkyl group having 1 to 3 carbon atoms,
R^{H} is CₘH₂ₘ₊₁, and
m is an integer of 1 to 3.

2. The method according to claim 1, wherein the compound containing a transition metal atom is a compound containing Mn, Co, Fe, Cu or Ni.

3. The method according to claim 1 or 2, wherein the compound containing a transition metal atom is a transition metal salt of nitric acid, a transition metal salt of sulfuric acid, a transition metal salt of acetic acid, a transition metal salt of carbonic acid, or a transition metal salt of phosphoric acid.

4. The method according to any one of claims 1 to 3, wherein the reaction of the compound represented by the formula (a) with oxygen is carried out in the presence of a carboxylic acid.

5. The method according to any one of claims 1 to 4, wherein the reaction of the compound represented by the formula (a) with oxygen is carried out in the presence of nitric acid, an alkali metal salt of nitric acid, or an alkaline-earth metal salt of nitric acid.

6. A method of producing a compound represented by the formula (b), which comprises reacting a compound represented by the formula (a) with oxygen in the presence of an alkali to obtain a compound represented by the formula (b); 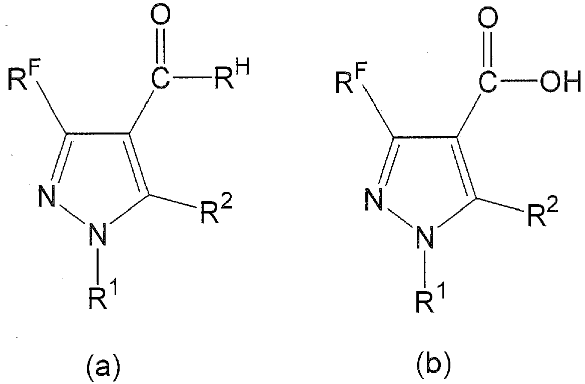 wherein
R¹ is an alkyl group having 1 to 3 carbon atoms,
R² is a hydrogen atom or a halogen atom,
R^{F} is a haloalkyl group having 1 to 3 carbon atoms,
R^{H} is CₘH₂ₘ₊₁, and
m is an integer of 1 to 3.

7. The method according to claim 6, which further comprises, after the reaction of the compound represented by the formula (a) with oxygen, reacting the resulting compound with an acid.

8. The method according to claim 6 or 7, wherein the reaction of the compound represented by the formula (a) with oxygen is carried cut in the presence of an organic solvent.

9. The method according to any one of claims 6 to 8, wherein the reaction of the compound represented by the formula (a) with oxygen is carried out in the presence of water.

10. The method according to any one of claims 6 to 9, wherein the alkali is sodium hydroxide or potassium hydroxide.
